# DEMANDE DE BREVET EUROPEEN

(11) **EP 3 598 997 A1**
(43) Date de publication de la demande: **29.01.2020**
(21) Numéro de dépôt: 19188393.3
(22) Date de dépôt: 25.07.2019
(51) Int. Cl.: A61Q 3/02, A61K 8/37, A61K 8/55, A61K 8/34, A61K 8/46, A61K 8/39, A61K 8/73, A61K 8/81, A61K 8/25, A61K 8/87

(54) **COMPOSITIONS AMÉLIORÉES COMME PRODUIT DE MAQUILLAGE APPLICABLE SUR LES ONGLES**

(30) Priorité: 27.07.2018 LU 100889
(71) Demandeur: International Lacquers S.A., 3225 Bettembourg (LU)
(72) Inventeur: VENTURA, Emmanuelle, 57000 Metz (FR); DEROSIER, Frédéric, 57680 Corny sur Moselle (FR); EDDOUMY, Fatima, 57840 Ottange (FR)
(74) Mandataire: Office Freylinger

(57) **Abrégé**

Composition de produit de maquillage pour les ongles comprenant au moins un solvant organique cosmétiquement acceptable, au moins un agent filmogène et un système de photoréticulation comprenant au moins un monomère et/ou oligomère (méth)acrylique et au moins un photoinitiateur ; la composition comprenant en outre au moins un constituant choisi parmi la silice pyrogénée hydrophile, la silice pyrogénée hydrophobe, les argiles modifiées et leurs mélanges ;.pour améliorer la brillance et la tenue de ladite composition.

## Description

### Domaine technique

La présente invention concerne de nouvelles compositions cosmétiques applicables sur les ongles en tant que produit de maquillage formant un film brillant et tenace.

### Etat de la technique

Les vernis à ongles sont généralement utilisés pour maquiller les ongles ainsi que les faux ongles. Les vernis à ongles dits «conventionnels» sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants. Les compositions de vernis à ongles peuvent s'appliquer aussi bien sur des ongles naturels que sur des faux-ongles.

Les compositions pour vernis à ongles peuvent être employées comme revêtement de base pour vernis (aussi appelée base-coat selon la terminologie anglo-saxonne), comme composition de maquillage des ongles ou vernis à ongles proprement dit, ou comme composition de finition (aussi appelée top-coat selon la terminologie anglo-saxonne). Les compositions dites revêtement de base correspondent à un revêtement que l'on applique directement au contact de l'ongle et/ou du faux-ongle afin de protéger l'ongle et de faciliter la pose et le retrait du vernis. Ces compositions dites de revêtement de base sont généralement incolores mais peuvent être colorées si un effet est recherché. Les compositions de finition s'appliquent sur le produit de maquillage des ongles proprement dit. Ces produits de finition sont généralement transparents et brillants afin d'apporter de la brillance et de la résistance à la couche sous-jacente. La présente invention concerne plus spécifiquement les compositions de maquillage des ongles, donc les vernis à ongles proprement dits.

Il existe dans le domaine des vernis à ongles en principe deux types de compositions qui se distinguent significativement par leur durée pendant laquelle elles peuvent rester sur l'ongle sans se détériorer significativement. Les vernis à ongles dits « conventionnels » (non réticulables) sont les bases dites « laque ou solvant » elles présentent souvent une adhésion de courte durée (typiquement 5 jours), mais on peut les retirer aisément. D'un autre côté, il existe les bases dites «gels UV» ou encore dites «soak-off» appliquées en institut spécialisé (uniquement professionnel) qui sont caractérisées par une très forte adhésion (3 semaines), mais peuvent induire des dommages importants de l'ongle (peeling) lors de leur retrait.

Les compositions dites « gels UV » sont des compositions liquides qui sous l'action d'un rayonnement lumineux provoquent des réactions de polymérisation et/ou de réticulation in situ aboutissant à des réseaux polymériques le plus souvent réticulés. De telles compositions photoréticulables sont communément appelées «gels UV».

Ce type de vernis à ongles est essentiellement constitué par des oligomères acryliques (de types acrylate, méthacrylate ou poly(éthylène glycol), de pigments, d'un photoinitiateur et de quantités faibles de solvant. Si la composition polymérique peut varier, leur modalité d'application reste la même. Sous l'effet d'un rayonnement UV ou visible, les photoinitiateurs amorcent une réaction de polymérisation des monomères et oligomères acryliques déposés sur l'ongle pour former un réseau polymérique complexe encapsulant les charges colorées. Il en résulte un film synthétique fin, compact et brillant capable de tenir plusieurs semaines sur l'ongle. L'intensité de réticulation est ajustée en fonction de la fonctionnalité du dépôt. Ainsi, la conception de faux ongles ou d'élongation de nature acrylique nécessite une densité de réticulation supérieure à un simple dépôt d'un vernis acrylique coloré. L'exceptionnelle adhésion et cohésion du film sur l'ongle ou sur un revêtement de base sont dues d'une part à la densité du maillage polymérique formé combiné à une diffusion partielle des oligomères et monomères acryliques à la surface de l'ongle ou du revêtement de base conduisant à une encapsulation partielle de la fibre de kératine présente à la surface de l'ongle ou des composants du revêtement de base. De ce fait, ce type de coating est généralement appliqué dans des instituts spécialisés à même de contrôler efficacement les méthodes de dépôt et le temps d'irradiation UV (« UV curing ») qui peuvent varier selon la nature des monomères et oligomères utilisés. Cette adhésion mécanique ou éventuellement chimique à la fibre de kératine peut induire un retrait du coating par peeling. Du fait de leur extrême stabilité, le retrait de ces bases acryliques ne se fait cependant pas sans dommage pour l'ongle. Les dommages induits par le retrait sont directement liés à la quantité de liens acryliques, entourant l'ongle et formés lors de la polymérisation.

Il est à noter qu'il existe sur le marché des gels UV pouvant être appliqués par les utilisateurs eux-mêmes. Pour favoriser leur tenue sur l'ongle, ils nécessitent cependant une étape de dépolissage de l'ongle afin de favoriser la tenue de la composition photoréticulée. De plus, ils nécessitent, lors du démaquillage, une étape de grattage de l'ongle par un outil métallique, une ponceuse électrique, ou une lime abrasive et/ou plonger les ongles pendant 5 à 10 minutes dans des solvants type acétone. Ces étapes peuvent considérablement et durablement endommager l'ongle. Les gels UV nécessitent également une étape de dégraissage pour éliminer l'effet collant de la surface.

Les vernis à ongles dits «conventionnels» (non réticulables) sont les bases dites « laque ou solvant » qu'on peut aisément retirer, mais elles présentent souvent une adhésion de courte durée (typiquement 5 jours). D'un autre côté, les bases dites «gels UV» ou encore dites «soak-off» appliquées en institut spécialisé (uniquement professionnel) sont caractérisées par une très forte adhésion (3 semaines), mais peuvent induire des dommages importants de l'ongle (peeling) lors de leur retrait.

La tenue dans le temps des vernis à ongles constitue donc un enjeu majeur. Les deux paramètres critiques caractérisant cette tenue dans le temps sont l'adhésion du vernis à ongles et la brillance.

Le challenge majeur dans le domaine des compositions pour vernis à ongles reste donc le développement de formulations non toxiques, à tenue de longue durée, mais néanmoins détachables facilement.

En conséquence, il existe un besoin de développer des vernis à ongles longue durée ne présentant pas les inconvénients des «gels UV» déjà présents sur le marché c'est-à-dire des vernis à ongles ne présentant pas de difficultés lors du retrait et n'induisant pas de dommages à l'ongle. Idéalement ces compositions permettent un maquillage de qualité dont la tenue et la brillance des compositions sont améliorées. En particulier, il existe une réelle demande de fournir des agents d'amélioration de la tenue et de la brillance de compositions de produit de maquillage des ongles compatibles avec tous les types de formulations de vernis à ongles, sans altérer (significativement) leurs autres propriétés. De préférence, ces compositions sont facilement démaquillables avec des démaquillants/dissolvants standards donc sans utilisation d'outils supplémentaires et sont, de plus, faciles à utiliser par les utilisateurs eux-mêmes.

### Objet de l'invention

Un objet de la présente invention est par conséquent de fournir une composition pour vernis à ongles en tant que produit de maquillage présentant une meilleure brillance et tenue de longue durée sur l'ongle tout en maintenant ses autres propriétés, à savoir : une bonne couvrance, ainsi qu'un temps de séchage et de durcissement courts.

### Description générale de l'invention

Afin de résoudre le problème mentionné ci-dessus, la présente invention propose, dans un premier aspect, une composition en tant que produit de maquillage pour les ongles comprenant au moins un solvant organique cosmétiquement acceptable, au moins un agent filmogène et un système de photoréticulation comprenant au moins un monomère et/ou oligomère (méth)acrylique et au moins un photoinitiateur.

Dans un deuxième aspect, l'invention propose également un kit (un nécessaire).

Dans un troisième aspect, l'invention propose un procédé de maquillage des ongles et/ou des faux-ongles.

Dans un quatrième aspect, l'invention concerne aussi l'utilisation d'une composition.

Les inventeurs ont découvert d'une manière surprenante que la combinaison d'une composition dite « conventionnelle » comprenant un agent filmogène, un solvant organique et un système de photoréticulation comprenant des oligomères et/ou monomères (méth)acryliques et un ou des photoinitiateurs, un constituant choisi parmi la silice pyrogénée hydrophile, la silice pyrogénée hydrophobe, les argiles modifiées et leurs mélanges, permettait d'améliorer très nettement non seulement la tenue et la brillance des compositions après application, mais également de faciliter énormément le démaquillage par comparaison aux gels UV.

En effet, les vernis à ongles dits « conventionnels » ont comme avantage de s'appliquer et de se démaquiller très facilement et rapidement. Or, ces vernis à ongles s'écaillent très vite. D'un autre côté, les compositions gels UV réticulables présentent, elles, une tenue longue durée mais restent difficiles à enlever avec comme risque de s'abimer les ongles. De plus, en raison de leurs résistances de longue durée, les utilisateurs ne peuvent modifier leur manucure à volonté.

La composition selon l'invention présente donc les avantages suivants:
- tenue de longue durée,
- la composition est facilement et rapidement démaquillable avec des démaquillants standards,
- les utilisateurs peuvent, au gré de leur envie, modifier leur manucure aussi aisément qu'avec des vernis à ongles « conventionnels ».

Une composition n'est pas juste une addition d'ingrédients facilement remplaçable par d'autres et il est probable que l'ajout ou la substitution d'un composant affecte négativement d'autres propriétés de la formulation. Par conséquent, l'avantage de la présente invention consiste à proposer des familles d'ingrédient compatibles entre eux et qui s'influencent mutuellement pour obtenir un effet de tenue et de brillance sans compromettre l'intégrité de la formulation.

Un avantage additionnel important de la présente invention est que les compositions de produit de maquillage pour les ongles, de préférence les formulations de vernis à ongles, à tenue et brillance améliorées peuvent comprendre les ingrédients habituellement utilisés par l'homme de l'art. L'homme de métier n'est donc pas obligé de reconsidérer tous les ingrédients et leurs effets, s'il souhaite utiliser les présents ingrédients d'amélioration de la tenue et de la brillance. Les présents ingrédients d'amélioration de la tenue et de la brillance présentent par conséquent une très bonne compatibilité tant avec les solvants, qu'avec les ingrédients courants dans le domaine.

De façon préférée, la composition en tant que produit de maquillage pour les ongles peut comprendre une quantité de résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique, ou de leurs combinaisons, comprise entre 0 % et 10% en poids sec, de préférence de 1 % à 10 % en poids sec, et de façon particulièrement préférée de 2 % à 8 % en poids sec par rapport au poids total de la composition.

Les compositions selon l'invention comprennent, en outre, de la silice pyrogénée qui se présente soit sous la forme de silice pyrogénée hydrophile, soit de silice pyrogénée hydrophobe, et/ou des argiles modifiées, comme par exemple le stéaralkonium hectorite ou le stéaralkonium bentonite soit encore d'un mélange des deux.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices sont: connues sous le nom commercial HDK (INCI: silica) comme par exemple, HDK N20, HDK N20D, HDK N20P, HDK N20ST, HDK S13, HDK T30, HDK T30P, HDK T40, HDK V15, ou sous le nom commercial Aerosil (INCI: silica) comme par exemple, Aerosil 200, Aerosil 300...

Il est possible de modifier la surface de ladite silice, par réaction chimique générant une diminution du nombre de groupes silanol. On peut notamment substituer des groupes silanol par des groupements hydrophobes : on obtient alors une silice hydrophobe. De telles silices sont connues sous le nom commercial HDK, HDK H18 (INCI : Silica Dimethyl Silylate), H13L (silica Dimethylsiloxy), ou sous le nom commercial Aerosil, Aerosil R202 (INCI: silica dimethicone silylate), Aerosil R972 (INCI: silica dimethyl silylate), Aerosil R812 (INCI: silica silylate), ....

La silice pyrogénée présente de préférence une surface spécifique (BET) comprise entre 20 et 500 m²/g. La composition peut comprendre de la silice pyrogénée en une quantité allant de 0,05 % à 5 % en poids, de préférence de 0,2 % à 1,3 % en poids, de manière particulièrement préférée 0,5 % à 1 % en poids par rapport au poids total de la composition.

La silice pyrogénée hydrophile ou hydrophobe est utilisée notamment comme agent thixotropique permettant de :
- moduler la viscosité de la composition,
- optimiser l'application de la formulation avec un pinceau. L'application est un paramètre essentiel pour ce type de composition cosmétique. L'application avec un pinceau doit permettre de former un film homogène en s'étalant de manière optimum sur l'ongle. Le paramètre d'étalement de la composition sur le support est capital pour l'utilisateur,
- permettre la tenue en suspension des particules solides au sein de la formulation.

Les argiles modifiées, dans le contexte de l'invention, sont généralement des argiles modifiées par des cations d'ammonium quaternaires, comme par les benzalkoniums, en l'occurrence par exemple le stéaralkonium hectorite, le stéaralkonium bentonite, ..., ou encore par des ions alkylammoniums, tels que le cétrimonium hectorite, le cétrimonium bentonite, ...

Les argiles modifiées seront de préférence utilisés de 0,1 à 4 % en poids sec par rapport au poids total de la composition.

La composition de produit de maquillage pour les ongles comprend un solvant organique. Ce solvant organique peut être choisi parmi les solvants usuels dans les vernis à ongles « conventionnels », de préférence dans le groupe constitué par le toluène, le xylène, l'acétate d'éthyle, l'acétate de butyle, l'acétate d'isobutyle, l'acétate de propyle, l'acétate de méthyle, une cétone, un ester, l'isopropanol, le butanol, le diacétone alcool, l'éthanol dénaturé (avec la méthyléthylcétone ou le diéthyléther), les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition de produit de maquillage pour les ongles. De façon préférée, la quantité de solvant organique présente dans la composition de produit de maquillage pour les ongles est comprise entre 20 % et 80 % en poids par rapport au poids total de la composition.

Avantageusement, le solvant organique est l'acétate de butyle et/ou l'acétate d'éthyle.

La composition de produit de maquillage pour les ongles selon l'invention comprend également un agent filmogène. Le ou les agent(s) filmogène(s) pouvant être compris dans la composition en tant que produit de maquillage pour les ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxy¬propyl¬cellulose. Préférentiellement, on choisira la nitrocellulose comme agent filmogène.

Un agent filmogène seul ou une combinaison d'agents filmogènes peut être utilisé(e) dans la composition de produit de maquillage pour les ongles dans les proportions allant de 4 % à 20 % en poids sec et de façon préférée de 8 % à 16 % en poids sec par rapport au poids total de la composition.

La composition de produit de maquillage pour les ongles comprend au moins un monomère et/ou oligomère (méth)acrylique qui est choisi parmi les monomères suivants, respectivement parmi les oligomères comprenant de 2 à 10 unités monomériques dont au moins un des monomères suivants:
- les méthacrylates de formule CH₂ = C(CH₃)-COOR₁,
   dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
   dans laquelle R₂ représente un groupe cycloalkyle en C₄ à C₁₂ tel que l'acrylate d'isobornyle ou un groupe tertio butyle,
- les (méth)acrylamides de formule : 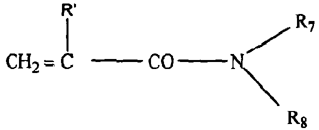 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges.

La quantité du au moins un monomère et/ou oligomère (méth)acrylique est de préférence comprise de 4 % à 60 % en poids sec, de manière particulièrement préférée de 5 % à 40 % en poids, sec par rapport au poids total de la composition.

La composition de produit de maquillage pour les ongles comprend également au moins un photoinitiateur qui est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine.

La quantité du au moins un photoinitiateur ou leur mélange est comprise de 0,2 à 10% en poids sec par rapport au poids total de la composition.

De manière avantageuse, le système de photoréticulation est utilisé en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons. De préférence, la résine est la résine de copolymère(s) par estérification de l'anhydride phtalique avec le néopentylglycol et/ou le triméthylolpropane et/ou le décanoate de glycidyle comprenant ou est constituée de résine de Polyester-23 (désignation INCI). Par conséquent, la présente invention propose également des compositions de revêtement de base comprenant en outre une ou plusieurs résine(s) choisie(s) parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/ acide phtalique, de préférence le Polyester-23.

La composition en tant que produit de maquillage pour les ongles peut aussi contenir au moins un plastifiant. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 0 % à 12 % en poids sec, de préférence 4 % à 8 % en poids sec par rapport au poids total de la composition. Le plastifiant est de préférence choisi parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide, etc.

En plus, de ou des résines selon l'invention, des résines supplémentaires (différentes) peuvent également être ajoutées dans la composition. En particulier, ces résines supplémentaires permettent d'augmenter le collant et l'adhérence du film sur l'ongle.

Les résines pouvant être utilisées sont les suivantes :
- les résines tosylamide/formaldéhyde ou résines toluènesulfonamide/ formaldéhyde,
- les résines tosylamide/époxy ou résines toluènesulfonamide/époxy,
- les copolymères de glycérine/acide phtalique,
- les copolymères styrène/acrylate/acrylonitrile,
- les polymères et copolymères d'acrylates,
- les copolymères d'acrylates et de styrène,
- le sucrose acétate isobutyrate,
- les résines polyvinylbutyral.

Pour colorer ou fournir des effets particuliers à la composition en tant que produit de maquillage pour les ongles, elle peut comprendre un ou plusieurs agents de coloration choisis parmi les pigments, les nacres, les glitters (paillettes) et/ou les particules métalliques. Ces agents de coloration fournissent l'aspect esthétique recherché par les utilisateurs de vernis à ongles. Ces agents de coloration peuvent produire des effets très différents tels que « irisé », « métallisé », « nacré », « miroir », « crème », « pailleté », craquelé », « mat », « néon », « fluorescent », « holographique », etc.

En particulier, les pigments utilisés peuvent être sélectionnés parmi le groupe constitué par le dioxyde de titane (CI 77891), l'oxyde de fer noir (CI 77499), l'oxyde de fer rouge (CI 77491), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters (paillettes) utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Selon un mode de réalisation de l'invention, la composition de produit de maquillage pour les ongles comprend une quantité d'agent de coloration comprise de 0,1 % à 30 % en poids sec et de préférence de 0,5 % à 14 % en poids sec par rapport au poids total de la composition.

La composition en tant que produit de maquillage pour les ongles peut en outre comprendre divers additifs choisis parmi les agents filmogènes, les résines, les modificateurs de surface et les agents dits « traitants ».

Parmi les additifs utilisables selon l'invention, on citera les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0,1 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05 % à 2 % en poids sec par rapport au poids total de la composition. Parmi ces additifs « traitants », on citera:
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Un autre objet de l'invention concerne un produit de maquillage pour les ongles comprenant une composition telle que décrite précédemment.

La présente invention concerne un kit comprenant au moins un revêtement de base, un produit de maquillage pour les ongles selon l'invention et (éventuellement) un produit de finition.

Un kit au sens de la présente invention signifie par exemple que les première, deuxième compositions et (éventuellement) la troisième composition sont commercialisées sous un même conditionnement, ou dans deux ou (éventuellement) trois conditionnements séparées sous un même emballage, ou dans deux ou (éventuellement) trois conditionnements séparés sous leur emballage respectif avec une indication d'utilisation combinée de la première composition et de la deuxième composition et (éventuellement) la troisième.

Selon un mode de réalisation particulier, les éléments du kit selon l'invention sont destinés à être appliqués sur les ongles et/ou faux-ongles selon le procédé de l'invention.

Selon un troisième aspect, l'invention propose un procédé de maquillage des ongles et/ou des faux-ongles comprenant au moins les étapes suivantes:
a) optionnellement, application sur un ongle ou un faux-ongle d'une première composition d'un kit conforme à l'invention, par laquelle on dépose un premier revêtement de base constitué d'au moins une couche d'une composition photoréticulable ou non, ce premier revêtement de base étant appliqué directement au contact de l'ongle ou du faux-ongle, et
b) optionnellement si le premier revêtement comprend une composition photoréticulable, exposition de l'ongle ou du faux-ongle revêtu obtenu à l'issue de l'étape a) à un rayonnement lumineux, UV ou visible,
c) application sur l'éventuel premier revêtement d'une ou plusieurs couches d'une composition de maquillage des ongles d'un kit conforme à l'invention, par laquelle on dépose un deuxième revêtement de produit de maquillage pour les ongles,
d) exposition de l'ongle ou du faux-ongle revêtu obtenu à l'issu de l'étape c) à un rayonnement lumineux, UV ou visible, si plusieurs couches sont appliquées, l'exposition de l'ongle ou du faux-ongle, est effectuée au moins après application de la dernière couche, éventuellement après chaque couche,
e) optionnellement, application sur le deuxième revêtement issu des étapes c) à d), d'un troisième revêtement constitué d'au moins une couche d'une troisième composition, de préférence un produit de finition photoréticulable ou non,
f) optionnellement si le troisième revêtement comprend une composition photoréticulable, exposition de l'ongle ou du faux-ongle revêtu obtenu à l'issu de l'étape e) à un rayonnement lumineux, UV ou visible.

Selon un mode de réalisation, le procédé de l'invention comprend en outre, avant l'étape b), une période de séchage du revêtement déposé à l'issue de l'étape a), dont la durée peut varier de 10 secondes à 20 minutes, typiquement de 1 minute à 10 minutes, voire de 5 minutes à 10 minutes. Ledit séchage est généralement effectué à l'air et à la température ambiante.

Les revêtements réticulés issus de la réticulation de l'étape b) et d) présentent une tenue dans le temps, en termes de résistance à l'écaillement et de brillance, significative et notamment à l'échelle d'au moins 7 à 15, voire 15 à 20 jours. Il s'avère ainsi résistant à l'eau, aux frottements et aux chocs, et ne présente pas d'usure ni d'écaillage significatifs dans ce délai.

Ces revêtements possèdent également une aptitude à se solubiliser ou à augmenter de volume, lorsqu'ils sont mis en contact avec un solvant de démaquillage usuel, par exemple l'acétone. Cette aptitude à se solubiliser ou à gonfler, manifestée par le revêtement réticulé, est précisément avantageuse pour son élimination lorsque celui-ci est appliqué en surface d'un ongle ou d'un faux-ongle. En effet, les revêtements peuvent être aisément éliminés par simple démaquillage à l'aide d'un dissolvant classique comme par exemple l'acétone.

Un quatrième aspect de l'invention concerne l'utilisation de silice hydrophile ou hydrophobe, d'argiles modifiées et/ou de leurs mélanges, d'un système de photoréticulation comprenant des oligomères et/ou monomères (méth)acrylique et un ou des photoinitiateurs ; de préférence en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons ; pour améliorer la tenue en durée des compositions cosmétiques après application en combinaison avec un démaquillage au dissolvant.

### Exemples

La composition en tant que produit de maquillage suivante a été préparée:

| Ingrédient | Quantité (% en poids) |
|---|---|
| acétate d'éthyle | 26,4733 |
| acétate de butyle | 23,0160 |
| diacétone alcool | 0,6600 |
| nitrocellulose | 9,7259 |
| alcool isopropylique | 4,1682 |
| polyester-23 | 4,5600 |
| acetyl tributyl citrate | 3,1386 |
| sucrose acétate isobutyrate | 0.4990 |
| copolymères de styrènes/acrylates | 1,00 |
| copolymères d'acrylates | 2,00 |
| stearalkonium bentonite | 1,100 |
| silica | 0,3899 |
| copolymères d'acide adipique/anhydride trimellitique/neopentyl glycols | 0,9702 |
| copolymères d'acide adipique/acide fumarique/diméthanol tricyclodécane | 0,6800 |
| (méth)acrylate d'isobornyle | 7,00 |
| PEG-4 trimethylolpropane triacrylate (et) pentaerythrityl tetramercaptopropionate | 10,00 |
| bis-trimethylbenzoyl phenylphosphine oxide | 1,00 |
| copolymère de Bis-hema poly(1,4-butanediol)-9/IDPI | 3,50 |
| pentaerythrityl tetraisostearate | 0,10 |
| acide phosphorique | 0,0189 |

La composition en tant que produit de maquillage pour les ongles, selon l'invention, a été préparée.

Concernant l'adhésion de la composition en tant que produit de maquillage pour les ongles, celle-ci est incomparable sur verre à celle d'une base classique non conforme à l'invention.

A l'issue du test du scotch, l'adhésion sur verre est mauvaise pour des bases classiques non conforme à l'invention, i.e., tout le film touché par le scotch s'enlève, contrairement aux bases réticulables selon l'invention pour lesquelles seuls les traits du quadrillage peuvent s'épaissir quelque peu.

En termes de dureté, qui est ici le paramètre le plus important à contrôler.

La dureté mesurée après 12h passées à l'étuve à 32°C se situe entre 170 et 200 secondes pour des bases classiques non conforme à l'invention alors qu'une dureté entre 100 et 170 secondes est ici visée car la réticulation va être efficace et il n'est pas souhaitable que la dureté augmente trop et crée ainsi du craquèlement sur les ongles.

Seulement 20 minutes après application, la dureté se situe déjà à 100 secondes pour une base classique non conforme à l'invention, contre 50 environ pour une base réticulable, pour lequel le réseau va mettre plus de temps à se former mais adhèrera rapidement à l'ongle. Au contraire, en exposant 1 min sous lampe LED, la dureté du film réticulable se trouve entre 100 et 180 secondes.

A l'application, les vernis sont collants mais après réticulation UV seulement (pas adaptable à la lumière visible), on obtient un film très lisse et très homogène ayant une très bonne adhésion à l'ongle.

Concernant la brillance, celle-ci est de 90-97 UB pour la base réticulable grâce aussi à l'aspect de surface décrit ci-dessus contre 80-88 UB pour la base classique.

De plus, les compositions de produit de maquillage pour les ongles présentent une tenue dans le temps, en terme de résistance à l'écaillement et de brillance significative et notamment à l'échelle d'au moins 7 à 15 jours. Les compositions s'avèrent ainsi résistantes à l'eau, aux frottements et aux chocs, et ne présentent pas d'usure ni d'écaillage significatifs dans ce délai.

Ces compositions se démaquillent avec un solvant de démaquillage usuel. En effet, un avantage de l'invention est que les compositions ne nécessitent plus de lime mais l'acétone peut simplifier le démaquillage. Le film, après nettoyage avec l'acétate d'éthyle, se décompose et se soulève de son substrat.

## Revendications

1. Composition de produit de maquillage pour les ongles comprenant au moins un solvant organique cosmétiquement acceptable, au moins un agent filmogène et un système de photoréticulation comprenant au moins un monomère et/ou oligomère (méth)acrylique et au moins un photoinitiateur ; la composition comprenant en outre au moins un constituant choisi parmi la silice pyrogénée hydrophile, la silice pyrogénée hydrophobe, les argiles modifiées et leurs mélanges.

2. Composition de produit de maquillage pour les ongles selon la revendication 1, **caractérisée en ce que** le système de réticulation y est compris en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons, la au moins une résine étant de préférence la résine de copolymère(s) par estérification de l'anhydride phtalique avec le néopentylglycol et/ou le triméthylolpropane et/ou le décanoate de glycidyle, de manière davantage préférée la résine de copolymère(s) par estérification de l'anhydride phtalique avec le néopentylglycol et/ou le triméthylolpropane et/ou le décanoate de glycidyle comprend ou est constituée de résine de Polyester-23 (désignation INCI).

3. Composition de produit de maquillage pour les ongles selon l'une quelconque des revendications précédentes, **caractérisée en ce que** la quantité de résine de copolymère(s) par estérification de l'anhydride phtalique avec le néopentylglycol et/ou le triméthylpropane et/ou le décanoate de glycidyle, est comprise entre 1 % et 10 % en poids sec et de façon préférée de 2 % à 8 % en poids sec par rapport au poids total de la composition.

4. Composition de produit de maquillage pour les ongles selon l'une quelconque des revendications précédentes, **caractérisée en ce que** la quantité de silice pyrogénée hydrophile ou hydrophobe est comprise entre 0,05 % et 5 % en poids sec et de façon préférée de 0,2 % à 1 % en poids sec par rapport au poids total de la composition.

5. Composition de produit de maquillage pour les ongles selon l'une quelconque des revendications précédentes, **caractérisée en ce que** les argiles modifiées sont choisis parmi argiles modifiées par des ions ammonium quaternaires, de préférence les argiles modifiées sont choisies parmi le stéaralkonium hectorite ou le stéaralkonium bentonite, la quantité d'argile(s) modifié(s) étant de préférence comprise jusqu'à 4% en poids par rapport au poids total de la composition pour les ongles.

6. Composition de produit de maquillage pour les ongles selon l'une quelconque des revendications précédentes, comprenant au moins un solvant organique choisi parmi le toluène, le xylène, l'acétate d'éthyle, l'acétate de butyle, l'acétate d'isobutyle, l'acétate de propyle, l'acétate de méthyle une cétone, un ester, l'isopropanol, le butanol le diacétone alcool, l'éthanol dénaturé avec la méthyléthylcétone ou le diéthyléther, les hydrocarbures linéaires ou cycliques, de préférence le cyclohexane, l'heptane ou n'importe quelle combinaison de ceux-ci, de préférence le solvant organique est l'acétate d'éthyle, l'acétate de butyle ou une combinaison de ceux-ci, la quantité de solvant organique étant de préférence comprise entre 20 % et 80 % en poids par rapport au poids total de la composition.

7. Composition de produit de maquillage pour les ongles selon l'une quelconque des revendications précédentes, **caractérisée en ce que** le au moins monomère et/ou oligomère (méth)acrylique est choisi parmi les monomères suivants, respectivement parmi les oligomères comprenant au moins un des monomères suivants:
- les méthacrylates de formule CH₂ = C(CH₃)-COOR₁,
dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que l'acrylate d'isobornyle ou un groupe tertio butyle,
- les (méth)acrylamides de formule : 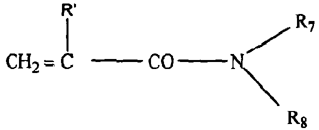 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges,
la quantité du au moins un monomère et/ou oligomère (méth)acrylique étant de préférence comprise de 4 % à 60 % en poids sec par rapport au poids total de la composition.

8. Composition de produit de maquillage pour les ongles selon l'une quelconque des revendications précédentes, **caractérisée en ce que** le au moins un photoinitiateur est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine, la quantité du photoinitiateur étant de préférence comprise de 0,2% à 10% en poids sec par rapport au poids total de la composition.

9. Composition de produit de maquillage pour les ongles selon l'une quelconque des revendications précédentes, **caractérisée en ce que** l'agent filmogène est choisi parmi la cellulose, un dérivé de cellulose, une résine vinylique, ou n'importe quelle combinaison de ceux-ci, de préférence l'agent filmogène est la nitrocellulose, la quantité d'agent filmogène étant de préférence comprise de 4 % à 20 % en poids sec et de façon préférée de 8 % à 16 % en poids sec par rapport au poids total de la composition.

10. Composition de produit de maquillage pour les ongles selon l'une quelconque des revendications précédentes, comprenant en outre un ou plusieurs plastifiants choisis parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide, ou leurs combinaisons, le ou les plastifiant(s) représentant de préférence de 0 % à 12 % en poids sec, de façon davantage préférée de 4 % à 8 % en poids sec par rapport au poids total de la composition.

11. Composition de produit de maquillage pour les ongles selon l'une quelconque des revendications précédentes, comprenant en outre un ou plusieurs additifs choisis parmi les résines, les modificateurs de surface et les agents dits « traitants » et/ou un ou plusieurs agents de coloration choisis parmi les pigments, les nacres, les glitters et/ou les particules métalliques.

12. Produit de maquillage pour les ongles comprenant une composition selon l'une quelconque des revendications précédentes.

13. Kit comprenant une composition de produit de maquillage selon l'une quelconque des revendications 1 à 11 ou un produit de maquillage pour les ongles selon la revendication 12, un revêtement de base et éventuellement un produit de finition.

14. Procédé de maquillage des ongles et/ou des faux-ongles facilement démaquillable comprenant les étapes suivantes:
(a) optionnellement, application sur un ongle ou un faux-ongle d'une première composition de revêtement de base d'un kit selon la revendication 13, par laquelle on dépose un premier revêtement de base constitué d'au moins une couche d'une composition photoréticulable ou non, ce premier revêtement de base étant appliqué directement au contact de l'ongle ou du faux-ongle, et
(b) optionnellement si le premier revêtement comprend une composition photoréticulable, exposition de l'ongle ou du faux-ongle revêtu obtenu à l'issue de l'étape a) à un rayonnement lumineux, UV ou visible,
(c) application sur l'éventuel premier revêtement d'une ou plusieurs couches d'une composition de maquillage des ongles selon l'une quelconque des revendications 1 à 11 ou d'une composition de maquillage d'un kit selon la revendication 12, par laquelle on dépose un deuxième revêtement de produit de maquillage pour les ongles,
(d) exposition de l'ongle ou du faux-ongle revêtu obtenu à l'issu de l'étape c) à un rayonnement lumineux, UV ou visible, si plusieurs couches sont appliquées, l'exposition de l'ongle ou du faux-ongle, est effectuée après application de la dernière couche,
(e) optionnellement, application sur le deuxième revêtement issu des étapes c) à d), d'un troisième revêtement constitué d'au moins une couche d'une troisième composition, de préférence un produit de finition photoréticulable ou non,
(f) optionnellement si le troisième revêtement comprend une composition photoréticulable, exposition de l'ongle ou du faux-ongle revêtu obtenu à l'issu de l'étape e) à un rayonnement lumineux, UV ou visible.

15. Utilisation de silice hydrophile ou hydrophobe, d'argiles modifiées et/ou de leurs mélanges et d'un système de photoréticulation comprenant des oligomères et/ou monomères (méth)acrylique et un ou des photoinitiateurs ; de préférence en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons ; pour améliorer la tenue en durée des compositions cosmétiques après application en combinaison avec un démaquillage au dissolvant.
